# NEUE EUROPÄISCHE PATENTSCHRIFT

(11) **EP 1 198 291 B2**
(45) Veröffentlichungstag und Bekanntmachung des Hinweises auf die Entscheidung über den Einspruch: **30.12.2009**
(45) Hinweis auf die Patenterteilung: 17.09.2003
(21) Anmeldenummer: 00929409.1
(22) Anmeldetag: 22.04.2000
(51) Int. Cl.: B01J 23/68, B01J 23/52, C07C 67/055, C07C 69/01

(54) **KATALYSATOR UND VERFAHREN ZUR HERSTELLUNG VON VINYLACETAT**
CATALYST AND METHOD FOR PRODUCING VINYL ACETATE
CATALYSEUR ET PROCEDE DE PRODUCTION D'ACETATE VINYLIQUE

(30) Priorität: 04.05.1999 DE 19920390
(43) Veröffentlichungstag der Anmeldung: 24.04.2002
(73) Patentinhaber: Celanese Chemicals Europe GmbH, 60439 Frankfurt am Main (DE)
(72) Erfinder: HERZOG, Bernhard, D-61440 Oberursel (DE); SCHÄFER, Axel, D-47169 Duisburg (DE); RENKEL, Karl-Heinz, D-46147 Oberhausen (DE); WANG, Tao, Corpus Christi, TX 78413 (US)
(74) Vertreter: Carpmaels & Ransford
(86) Internationale Anmeldenummer: PCT/EP2000/003656
(87) Internationale Veröffentlichungsnummer: WO 2000/066261

(56) Entgegenhaltungen:
- EP-A- 0 723 810
- EP-A- 0 967 009
- EP-A- 0 967 009
- DE-A- 1 811 834
- DE-A- 2 811 211
- GB- - 1 146 791
- US-A- 3 637 819
- US-A- 3 903 139

## Beschreibung

Die vorliegende Erfindung betrifft einen Katalysator, der Palladium und/oder dessen Verbindungen, Gold und/oder dessen Verbindungen, Alkalimetallverbindungen und Vanadium und/oder dessen Verbindungen enthält, und seine Verwendung zur Herstellung von Vinylacetat aus Essigsäure, Ethylen und Sauerstoff oder Sauerstoff enthaltenden Gasen.

Es ist bekannt, daß man in der Gasphase Ethylen mit Essigsäure und Sauerstoff oder sauerstoffhaltigen Gasen an Palladium/Gold/Alkalimetall enthaltenden Festbett-Katalysatoren zu Vinylacetat umsetzen kann.

Die Palladium/Gold/Alkalimetall enthaltenden Katalysatoren weisen in der Regel eine besondere Edelmetallverteilung auf, bei der die Edelmetalle in einer Schale auf den Trägerteilchen vorliegen, während der Kern der Teilchen weitgehend frei von Edelmetallen ist. Die Edelmetallverteilung in Schalenform erreicht man durch imprägnieren und anschließendes Ausfällen der Edelmetalle mit alkalischen Verbindungen. Katalysatoren mit dieser Edelmetallverteilung zeigen eine gute Aktivität und bilden im allgemeinen wenig Kohlendioxid und Ethylacetat. Ein weiteres Merkmal dieser Katalysatoren besteht darin, daß sich bei Verwendung dieser Katalysatoren nur geringe Mengen Hochsieder bilden. Obwohl diese Mengen nur gering sind, stellen sie ökologisch und verfahrenstechnisch ein Problem dar. Solche Hochsieder sind z.B. Ethylidendiacetat, Ethylenglykol und dessen Acetate oder Diacetoxyethylene.

US-A-3 775 342 offenbart ein Verfahren zur Herstellung von Palladium, Kalium und Gold enthaltenden Katalysatoren durch Imprägnierung eines Trägers mit einer Lösung von Palladium- und Goldsalzen, durch anschließende Behandlung mit einer Alkalilösung, die dazu führt, daß sich wasserunlösliche Palladium- und Goldverbindungen auf dem Träger niederschlagen, und durch anschließende Reduktion der Metallverbindungen zu den entsprechenden Edelmetallen. Eine Behandlung des Trägermaterials mit einer Alkalimetallacetatlösung kann vor oder nach dem Reduktionsschritt erfolgen.

US-A-4 048 096 lehrt ein Verfahren zur Herstellung von Palladium, Kalium und Gold enthaltenden Katalysatoren, in dem zunächst das Trägermaterial mit einer wäßrigen Lösung, die eine Mischung aus Palladium- und Goldsalzen enthält, imprägniert wird. Dabei entspricht das Volumen der Imprägnierlösung dem Porenvolumen des Trägermaterials. Anschließend wird der feuchte Träger mit einer wäßrigen Alkalilösung, z.B. mit einer wäßrigen Natriummetasilikatlösung vollständig überschichtet und bei Raumtemperatur über 12 Stunden ruhend belassen. Auf diese Weise werden die Metallsalze in wasserunlösliche Verbindungen überführt und so auf dem Trägermaterial fixiert. Durch nachfolgende Behandlung mit einem Reduktionsmittel werden die Palladium- und Goldverbindungen zu den entsprechenden Metallen reduziert. Dazu wird beispielsweise eine wäßrige Hydrazinlösung unter leichter Bewegung hinzugegeben und der Ansatz nach Zugabe über 4 Stunden ruhend belassen. Nach Wäsche und Trocknung wird das mit Palladium und Gold beladene Trägermaterial mit einer Alkalimetallacetatlösung behandelt und erneut getrocknet. Der erhaltene Katalysator weist eine Schalenstruktur auf, in der Palladium und Gold in einer Schalendicke von etwa 0,5 Millimetern über die Oberfläche des Trägermaterials verteilt sind.

In dem in US-A-5 332 710 offenbarten Verfahren zur Herstellung eines Palladium, Gold und Kalium enthaltenden Schalenkatalysators wird der mit einer wäßrigen Palladium- und Goldsalzlösung imprägnierte Träger in eine Natriumhydroxid oder Kaliumhydroxid enthaltende wäßrige Fixierlösung eingetaucht und darin wenigstens 0,5 h lang bewegt. Die offenbarte Fixierungstechnik ist dadurch charakterisiert, daß der mit der Fixierlösung vollständig bedeckte Träger ab dem Beginn der Behandlung mit der Fixierlösung rotierend bewegt wird.

US 3,637,819 offenbart einen Prozeß zur Herstellung von Vinylacetat in der Gasphase in Gegenwart eines Trägerkatalysators, der Palladiumacetat, Alkalimetallacetat und eine oder mehrere Vanadiumverbindungen auf einem Träger niedergeschlagen enthält. Dabei wird die getrocknete Katalysatorvorstufe nach dem Imprägnierschritt bestrahlt.

Überraschenderweise wurde nun gefunden, daß der Zusatz von Vanadium und/oder dessen Verbindungen die Hochsieder-Selektivität des Katalysators deutlich verbessert. Unter der Hochsieder-Seiektivität versteht man das Verhältnis der in der Vinylacetatsynthese gebildeten Menge an Hochsiedern zu der Menge an umgesetztem Ethylen. Unter Hochsiedem werden unter anderem die zuvor erwähnten Verbindungen verstanden.

Ein Gegenstand der Erfindung ist demgemäß ein Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, Gold und/oder dessen Verbindungen sowie Alkalimetallverbindungen auf einem Träger enthält, **dadurch gekennzeichent, daß** der Katalysator zusätzlich 0,01 Gew.-% bis 1 Gew.-% Vanadium und/oder dessen Verbindungen, berechnet als Element und bezogen auf die Gesamtmasse des Katalysators, enthält.

Ein weiterer Gegenstand der Erfindung ist ein Katalysator, der Palladium und/oder dessen Verbindungen, Gold und/oder dessen Verbindungen sowie Alkalimetallverbindungen auf einem Träger enthält, **dadurch gekennzeichnet, daß** der Katalysator zusätzlich 0,01 Gew.-% bis 1 Gew.-% Vanadium und/oder dessen Verbindungen, berechnet als Element und bezogen auf die Gesamtmasse des Katalysators, enthält.

Die Herstellung der erfindungsgemäßen Katalysatoren erfolgt vorzugsweise, indem man:
(1) den Träger mit einer löslichen Vanadiumverbindung imprägniert und anschließend trocknet;
(2) den vorbehandelten Träger mit löslichen Palladium- und Goldverbindungen imprägniert;
(3) die löslichen Palladium- und Goldverbindungen durch eine alkalisch reagierende Lösung auf dem Träger in unlösliche Verbindungen überführt;
(4) die unlöslichen Palladium- und Goldverbindungen auf dem Träger durch ein Reduktionsmittel reduziert;
(5) den Träger wäscht und anschließend trocknet;
(6) den Träger mit einer löslichen Alkalimetallverbindung imprägniert; und
(7) den Träger abschließend bei höchstens 150°C trocknet.

Die Arbeitsschritte (2) bis (7) sind z.B. aus US-A-3 775 342; US-A-4 048 096 und US-A-5 332 710 bekannt.

Neben der Imprägnierung des Trägers mit den löslichen Vanadium-, Palladium- und Gold- sowie Alkalimetall-verbindungen lassen sich auch andere dem Fachmann vertraute Techniken anwenden, um die katalytisch aktiven Substanzen auf dem Träger aufzubringen, wie zum Beispiel mehrmaliges Aufdampfen, Aufsprühen oder Tauchen, gegebenenfalls unter Anwendung von Ultraschall.

Ebenfalls ist es möglich, die Arbeitsschritte (1) und (2) zu vertauschen, d.h. den Träger zunächst mit einer Palladium- und Goldverbindungen enthaltenden Lösung zu imprägnieren und nach erfolgter Trocknung eine Vanadiumverbindung auf den behandelten Träger aufzubringen.

Als Träger eignen sich die bekannten inerten Trägermaterialien wie Kieselsäure, Aluminiumoxid, Alumosilikate, Silikate, Titanoxid, Zirkonoxid, Titanate, Siliciumcarbid und Kohle. Besonders geeignet sind Träger dieser Art mit einer spezifischen Oberfläche von 40 bis 350 m²/g (gemessen nach der BET-Methode) und einem mittleren Porenradius von 50 bis 2000 Å (Ängström) (gemessen mit Quecksilber-Porosimetrie), vor allem Kieselsäure (SiO₂) und SiO₂-Al₂O₃-Gemische. Diese Träger können in jedweder Form, wie z.B. in Form von Kugeln, Tabletten, Ringen, Sternen oder anders geformten Teilchen benutzt werden, deren Durchmesser bzw. deren Länge und Dicke im allgemeinen bei 3 bis 9 mm liegt.

Als Träger können z.B. aerogenes SiO₂ oder ein aerogenes SiO₂-Al₂O₃-Gemisch gewählt werden, die beispielsweise durch Flammenhydrolyse von Siliciumtetrachlorid oder eines Siliciumtetrachlorid-Aluminiumtrichlorid-Gemisches in einer Knallgasflamme hergestellt werden können (US-A-3 939 199).

Für die in der Arbeitsweise nach Schritt (1) aufzubringende Vanadiumverbindung verwendet man vorzugsweise ein Vanadiumsalz, wie Vanadylsalze, Vanadate und Isopolyvanadate. Besonders bevorzugt setzt man Vanadylsalze, wie Chloride, Sulfate, Oxalate, Acetate und Acetylacetonate ein. Man kann auch mehrere Vanadiumsalze und/oder Vanadylsatze aufbringen , im allgemeinen bringt man aber genau ein Vanadiumsalz oder ein Vanadylsalz auf.

Die in der Arbeitsweise nach Schritt (2) jeweils aufzubringenden Elemente Palladium und Gold können vorzugsweise in Form von Salzlösungen einzeln in unabhängiger Reihenfolge oder gemeinsam aufgebracht werden, vorzugsweise verwendet man eine einzige Lösung, die diese aufzubringenden Elemente in Form von Salzen enthält. Besonders bevorzugt ist der Einsatz einer einzigen Lösung, die von jedem dieser aufzubringenden Elemente genau ein Salz enthält.

Dabei muß aber im Fall störender Anionen, wie z.B. bei Chloriden, sichergestellt werden, daß diese Anionen vor dem Einsatz des Katalysators weitgehend entfernt werden. Dies geschieht durch Auswaschen des dotierten Trägers, z.B. mit Wasser, nachdem beispielsweise das als Chlorid aufgebrachte Palladium und Gold in eine unlösliche Form überführt wurden, etwa durch die Fixierung mit alkalisch reagierenden Verbindungen und/oder durch Reduktion (Arbeitsschritte (3) und (4)).

Als Salze von Palladium und Gold sind alle Salze geeignet, die löslich sind. Besonders geeignet sind Chloride, Chlorokomplexe und Carboxylate, vorzugsweise die Salze der aliphatischen Monocarbonsäuren mit 2 bis 5 Kohlenstoffatomen, beispielsweise das Acetat, Propionat oder Butyrat. Weiter sind beispielsweise geeignet das Nitrat, Nitrit, Oxidhydrat, Oxalat, Acetylacetonat oder Acetoacetat. Wegen der guten Löslichkeit und Verfügbarkeit sind besonders die Chloride und Chlorokomplexe von Palladium und Gold bevorzugte Palladium- und Goldsalze.

Als Alkalimetallverbindung wird vorzugsweise mindestens eine Natrium-, Kalium-, Rubidium- oder Caesiumverbindung eingesetzt, insbesondere eine Kaliumverbindung. Geeignet als Verbindungen sind vor allem Carboxylate, insbesondere Acetate und Propionate. Geeignet sind auch Verbindungen, die unter den Reaktionsbedingungen in das Alkaliacetat übergehen, wie etwa das Hydroxid, das Oxid oder das Carbonat.

Als Lösungsmittel für die Palladium-, Gold-, Alkalimetall- und Vanadiumverbindungen sind solche geeignet, in denen die gewählten Verbindungen löslich sind und die nach der Imprägnierung leicht wieder durch Trocknung zu entfernen sind. Wählt man Palladium-, Gold-, Alkalimetall- und Vanadiumalze, so eignen sich für die Acetate und Acetylacetonate vor allem unsubstituierte Carbonsäuren mit 2 bis 10 Kohlenstoffatomen, wie Essigsäure, Propionsäure, n- und iso-Buttersäure und die verschiedenen Valeriansäuren. Wegen ihrer physikalischen Eigenschaften und auch aus wirtschaftlichen Gründen wird Essigsäure unter den Carbonsäuren bevorzugt. Für die Chloride, Chloro-, Acetatokomplexe und Acetylacetonate ist vor allem Wasser geeignet. Die zusätzliche Verwendung eines weiteren Lösungsmittels ist dann zweckmäßig, wenn die Salze in der Essigsäure oder im Wasser nicht genügend löslich sind. So läßt sich z.B. Palladiumchlorid in einer wäßrigen Essigsäure wesentlich besser lösen als in Eisessig. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, aber auch Kohlenwasserstoffe wie Benzol.

Wenn im folgenden im allgemeinen über "die Lösung der Salze" gesprochen wird, so gilt sinngemäß dasselbe für den Fall, daß mehrere Lösungen der Reihe nach eingesetzt werden, die jeweils nur einen Teil der insgesamt aufzubringenden Salze enthalten, wobei sich die einzelnen Teile zur Gesamtmenge der Salze ergänzen, die auf den Träger aufgebracht werden sollen.

Zur Durchführung der Arbeitsweise nach Schritt (1) und (2) wird die Lösung der Salze auf die Trägerteilchen aufgebracht, indem man diese ein- oder mehrmals mit dieser Lösung tränkt, wobei das gesamte Volumen der Lösung auf einmal oder in zwei oder mehreren Teilvolumina unterteilt eingesetzt wird. Zweckmäßig ist es jedoch, das gesamte Volumen der Salzlösung auf einmal zu verwenden, so daß durch einmaliges Tränken die Trägerteilchen mit der gewünschten Menge an den aufzubringenden Elementen imprägniert werden, wobei sich die Trocknung sofort anschließen kann. Beim Tränken der Reihe nach mit mehreren Teilvolumina wird nach jedem Tränken sofort getrocknet.

Die "sofortige" Trocknung bedeutet dabei, daß zügig mit der Trocknung der getränkten Teilchen begonnen werden muß. Dabei reicht es im allgemeinen, wenn nach dem Ende einer Tränkung spätestens nach 1/2 Stunde mit der Trocknung der Teilchen begonnen wird.

Die Tränkung der Trägerteilchen mit der Lösung der aufzubringenden Salze wird so vorgenommen, daß die Trägerteilchen mit der Lösung überschichtet und gegebenenfalls überschüssige Lösung dann abgegossen oder abfiltriert wird. Mit Rücksicht auf Lösungsverluste ist es vorteilhaft, nur die dem integralen Porenvolumen des Katalysatorträgers entsprechende Menge an Lösung einzusetzen, daß also das Volumen der Imprägnierlösung vorzugsweise 98-100% des Porenvolumens des Katalysatorträgers entspricht.

Es ist zweckmäßig, während des Tränkens die Trägerteilchen innig zu durchmischen, beispielsweise in einem sich drehenden oder bewegten Kolben oder einer Mischtrommel, wobei sich die Trocknung sofort anschließen kann. Die Drehgeschwindigkeit oder Intensität der Bewegung muß einerseits groß genug sein, um eine gute Durchmischung und Benetzung der Trägerteilchen zu gewährleisten, andererseits darf sie nicht so hoch sein, daß es zu wesentlichem Abrieb am Trägermaterial kommt.

Durch die Behandlung der gemäß der Arbeitsschritte (1) und (2) getränkten Trägerteilchen mit einer alkalisch reagierenden Lösung werden die Salze der aufgebrachten Elemente in wasserunlösliche Verbindungen überführt und so auf der Trägeroberfläche fixiert (Arbeitsschritt (3)).

Als Fixierlösungen lassen sich beispielsweise wäßrige alkalisch reagierende Lösungen verwenden. Beispiele für derartige Lösungen sind wäßrige Lösungen von Alkalimetallsilikaten, Alkalimetallcarbonaten und - hydrogencarbonaten, Alkalimetallhydroxiden oder Alkalimetallboraten.

Bevorzugt ist eine wäßrige Lösung der Alkalihydroxide, insbesondere Kalium- oder Natriumhydroxid. Auch wäßrige Lösungen, die Borverbindungen enthalten, können als alkalisch reagierende Lösungen verwendet werden. Hierbei sind besonders wäßrige Lösungen von Borax (Natriumtetraborat-Dekahydrat), Kaliumtetraborat oder Mischungen aus Alkalilauge und Borsäure geeignet. Die alkalische Lösung kann Puffereigenschaften aufweisen.

Die Menge der in der Fixierlösung enthaltenden alkalisch reagierenden Verbindung ist zweckmäßigerweise so bemessen, daß sie für die stöchiometrische Umsetzung der aufgebrachten, löslichen Palladium- und Goldverbindungen zu wasserunlöslichen Verbindungen mindestens ausreichend sind.

Es kann aber auch ein Überschuß an der in der Fixierlösung enthaltenen alkalisch reagierenden Verbindung angewandt werden, der Überschuß liegt im allgemeinen bei der 1- bis 10-fachen Menge, bezogen auf die stöchiometrisch erforderliche Menge.

Das Volumen der Fixierlösung ist wenigstens so zu bemessen, daß es ausreicht, den imprägnierten Träger vollständig mit der Fixierlösung zu bedecken. Vorzugsweise erfolgt die Fixierung nach der aus US-A-5,332,710 bekannten Technik, auf die hiermit direkt Bezug genommen wird ("incorporation by reference"). Diese Technik ist dadurch charakterisiert, daß der mit der Fixierlösung vollständig bedeckte Träger ab dem Beginn der Behandlung mit der Fixierlösung rotierend bewegt wird.

Jede Art von Rotation oder ähnlicher Behandlung, die die Trägerteilchen in Bewegung hält, kann benutzt werden, da die genaue Art und Weise nicht kritisch ist. Wichtig ist jedoch die Intensität der Bewegung. Diese soll ausreichen, die gesamte Oberfläche der imprägnierten Träger gleichmäßig mit der alkalischen Fixierlösung zu benetzen.

Danach beläßt man den behandelten Träger bis zu 16 Stunden bei Raumtemperatur ruhend in der Fixierlösung, um sicherzustellen, daß die aufgebrachten Palladium- und Goldverbindungen vollständig auf dem Katalysatorträger in Form von wasserunlöslichen Verbindungen ausgefällt werden

Die Umsetzung auf dem Träger kann bei Raumtemperatur aber auch bei erhöhter Temperatur, z.B. bei 70°C durchgeführt werden.

Die Vorgehensweise bei der anschließenden Reduktion der unlöslichen Palladium- und Goldverbindungen (Arbeitschritt 4) hängt davon ab, ob man mit einem gasförmigen oder flüssigen Reduktionsmittel arbeitet.

Falls man mit einem flüssigen Reduktionsmittel arbeitet, gibt man nach beendeter Fixierung das flüssige Reduktionsmittel hinzu, gegebenfalls erst nach dem Abgießen der überstehenden Fixierlösung.

Die Reduktion erfolgt bei einer Temperatur von 0°C bis 90°C, vorzugsweise von 15 bis 25°C. Als Reduktionsmittel lassen sich beispielsweise wäßrige Lösungen von Hydrazin, Ameisensäure oder Alkaliborhydriden, vorzugsweise Natriumborhydrid, verwenden.

Nach der Reduktion muß der behandelte Katalysatorträger zur Entfernung störender Verbindungen, z.B. zur Entfernung von Chloridresten, die vom Imprägnierungsschritt herrühren, und die durch die Fixierung und Reduktion der Edelmetalle freigesetzt werden, mehrmals gewaschen werden(Arbeitsschritt (5)).

Dazu wird der behandelte Träger bei Raumtemperatur mit der Waschflüssigkeit, vorzugsweise mit fließendem, demineralisiertem Wasser kontinuierlich gewaschen, bis störende Anionen, z.B. Chloride, entfernt sind. Mit dem Waschvorgang lassen sich auch Reste des verwendeten Reduktionsmittels entfernen.

Anschließend wird die feuchte Katalysatorvorstufe bei Temperaturen von höchsten 150°C getrocknet (Arbeitsschritt 5).

Arbeitet man in Gegenwart eines gasförmigen Reduktionsmittels, so wird nach beendeter Fixierung zunächst die überstehende Fixierlösung abgegossen. Anschließend empfiehlt es sich, den nach dem Fixierungsschritt erhaltenen, behandelten Träger vor dem Reduktionsschritt zu waschen, um die auf dem behandelten Träger enthaltenen löslichen Verbindungen, z.B. die aus dem Fixierungsschritt freigesetzten Alkalimetallchloride und einen gegebenenfalls vorhandenen Überschuß an der in der Fixierlösung enthaltenen alkalisch reagierenden Verbindung zu entfernen.

Dazu wird der behandelte Träger bei Raumtemperatur mit der Waschflüssigkeit, vorzugsweise mit fließendem, demineralisiertem Wasser kontinuierlich gewaschen. Die Wäsche wird fortgesetzt, bis störende Anionen, z.B. Chloride vom Träger weitgehend entfernt sind. Anschließend ist es zweckmäßig, den feuchten imprägnierten Katalysatorträger vor der mit einem gasförmigen Reduktionsmittel durchgeführten Reduktion zu trocknen. Die Trocknung wird dabei bei Temperaturen bis höchstens 150°C durchgeführt.

Die anschließende Reduktion erfolgt bei einer Temperatur im allgemeinen zwischen 40 und 260°C, vorzugsweise zwischen 70 und 200°C. Im allgemeinen ist es zweckmäßig, für die Reduktion ein mit Inertgas verdünntes Reduktionsmittel zu verwenden, das 0,01 bis 50 Vol.-%, vorzugsweise 0,5 bis 20 Vol.-% Reduktionsmittel enthält. Als Inertgas kann beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen oder andere Olefine in Frage.

Unabhängig davon, ob man die Reduktion in Gegenwart eines gasförmigen Reduktionsmittels oder unter Verwendung eines flüssigen Reduktionsmittels vornimmt ist die Menge des Reduktionsmittels nach der Menge der Edelmetalle zu bemessen; das Reduktionsäquivalent soll mindestens das einfache an Oxidationsäquivalent betragen, jedoch schaden größere Mengen Reduktionsmittel nicht.

Bei dem Reduktionsschritt ist es wesentlich, die Reduktionsbedingungen so zu wählen, daß eine Reduktion der fixierten wasserunlöslichen Edelmetallverbindungen zu den entsprechenden Edelmetallen erfolgt. Hingegen ist es unwesentlich, ob unter den gewählten Reduktionsbedindungen auch das in Vanadiumverbindungen vorliegende Vanadium in elementares Vanadium überführt wird, da dies für die Eignung der erfindungsgemäßen Katalysatoren zur Vinylacetatherstellung nicht kritisch ist.

Die nach dem Reduktions- und gegebenenfalls Trocknungsschritt vorliegende Katalysatorvorstufe wird gemäß Arbeitsschritt (6) mit einer Lösung einer Alkalimetallverbindung ein- oder mehrmals behandelt, vorzugsweise imprägniert, wobei das gesamte Volumen der Lösung auf einmal oder in Teilvolumina unterteilt eingesetzt wird. Zweckmäßig ist es jedoch, das gesamte Volumen der Lösung auf einmal zu verwenden, so daß durch einmaliges Tränken die Trägerteilchen mit den gewünschten Mengen an aufzubringender Alkalimetallverbindung imprägniert werden. Das Lösungsvolumen der Alkalimetallverbindung liegt bei dem einmaligen oder bei einem mehrfachen Imprägnieren im allgemeinen zwischen 60 und 110 %, vorzugsweise zwischen 80 und 100 % des Porenvolumens.

Die Lösung der Alkalimetallverbindung kann auch durch ein- oder mehrmaliges Aufsprühen, Aufdampfen oder Tauchen auf der Katalysatorvorstufe aufgebracht werden.

Nach dem Behandeln mit einer Lösung einer Alkalimetallverbindung wird die Katalysatorvorstufe abschließend bei höchstens 150°C getrocknet (Arbeitsschritt (7)).

Die Alkalimetaliverbindung wird in einer solchen Menge verwendet, so daß der fertige Katalysator nach dem Trocknen 0,1 bis 10 Gew.-% an Alkalimetall enthält.

Die Trocknungsschritte der behandelten Katalysatorträger oder der Katalysatorvorstufen erfolgen in einem Heizluftstrom oder in einem Inertgasstrom, beispielsweise in einem Stickstoff- oder Kohlendioxidstrom. Die Temperatur bei der Trocknung sollte dabei im allgemeinen 60 bis 150°C, vorzugsweise 100 bis 150°C betragen. Gegebenenfalls wird dabei unter vermindertem Druck, im allgemeinen bei 0,01 MPa bis 0,08 MPa getrocknet.

Die fertigen Palladium, Gold, Alkalimetall und Vanadium enthaltenden Schalenkatalysatoren weisen folgende Metallgehalte auf:

| | |
|---|---|
| Palladiumgehalt | im allgemeinen 0,5 - 2,0 Gew.-%, |
| | vorzugsweise 0,6 - 1,5 Gew.-%; |
| Goldgehalt | im allgemeinen 0,2 - 1,3 Gew.-%, |
| | vorzugsweise 0,3 - 1,1 Gew.-%; |
| Alkalimetallgehalt | im allgemeinen 0,3 - 10 Gew.-%, |

Vorzugsweise verwendet man Kalium.

| | |
|---|---|
| Kaliumgehalt | im allgemeinen 0,5 - 4,0 Gew.-%, |
| | vorzugsweise 1,5 - 3,0 Gew.-%; |
| Vanadiumgehalt | 0,01 - 1 Gew.-%, |
| | vorzugsweise 0,05 - 0,5 Gew.-%. |

Die angegebenen Prozentzahlen betreffen stets die im fertigen Katalysator anwesenden Mengen der Elemente Palladium, Gold, Alkalimetall und Vanadium, bezogen auf die Gesamtmasse des Katalysators (aktive Elemente plus Anionen plus Trägermaterial).

Bei den erfindungsgemäßen Katalysatoren sind die Edelmetalle in einer Schale auf dem Trägerteilchen aufgebracht.

Die Herstellung des Vinylacetats erfolgt im allgemeinen durch Leiten von Essigsäure, Ethylen und Sauerstoff enthaltenden Gasen bei Temperaturen von 100 bis 220°C, vorzugsweise 120 bis 200°C, und bei Drücken von 0,1 bis 2,5 MPa, vorzugsweise 0,1 bis 2,0 MPa, über den fertigen Katalysator, wobei nichtumgesetzte Komponenten im Kreis geführt werden können. Unter Umständen ist auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Mit Hilfe der erfindungsgemäßen Katalysatoren können die Aktivität und Selektivität verbessert werden, wodurch insbesondere die Hochsiederbildung deutlich verringert wird. Hochsieder sind insbesondere die eingangs erwähnten Verbindungen, die sowohl ökologisch als auch verfahrenstechnisch ein Problem darstellen.

Das unter Verwendung der erfindungsgemäßen Katalysatoren durchgeführte Vinylacetatverfahren liefert somit auch eine höhere Ausbeute an Vinylacetat, wodurch die Aufarbeitung des erhaltenen Rohvinylacetats erleichtert wird, da der Vinylacetatgehalt im Reaktorausgangsgas höher ist, was weiterhin zu einer Energieersparnis im Aufarbeitungsteil führt. Eine geeignete Aufarbeitung wird z.B. in der US-A-5 066 365 beschrieben.

Die folgenden Beispiele sollen die Erfindung erläutern, schränken sie aber nicht ein. Die Prozentangaben der Elemente Palladium, Gold, Kalium und Vanadium sind Gewichtsprozente, bezogen auf die Gesamtmasse des fertigen Katalysators.

### Beispiele

Für sämtliche Beispiele dienten 7mm-Pellets aus KA-160 Kieselsäure auf Bentonitbasis der Firma Süd-Chemie als Trägermaterial.

### Beispiel 1

0,17 g (0,0007 mol) Vanadylacetylacetonat wurden in 32 ml demineralisiertem Wasser gelöst und auf 52,4 g Trägermaterial aufgebracht. Danach wurde der behandelte Träger 2 Stunden bei 100°C getrocknet.

2,15 g (0,0066 mol) K₂PdCl₄ und 0,77 g (0,002 mol) KAuCl₄ wurden zusammen eingewogen und in 32 ml demineralisiertem Wasser gelöst. Diese Lösung wurde unter leichter Bewegung auf den vorbehandelten Träger vollständig aufgetragen. im Anschluß wurde bei 100°C für 2 Stunden getrocknet.

Zur Ausbildung einer Edelmetallschale und der Überführung der Edelmetallsalze in unlösliche Verbindungen wurde der vorbehandelte Träger mit einer Lösung von 1,74 g (0,031 mol) Kaliumhydroxid in 32 ml demineralisiertem Wasser übergossen. Die Reaktionsmischung wurde zur Vervollständigung der Reaktion über 14 Stunden ruhengelassen und danach mit demineralisiertem Wasser chloridfrei gewaschen. Die Überprüfung auf Chloridfreiheit erfolgte mittels AgNO₃-Nachweis auf Chloridionen in wässriger Lösung. Im Anschluß wurde bei 100°C für 2 Stunden getrocknet.

Anschließend erfolgte die Reduktion der Edelmetalle mit verdünntem Ethylen (5 Vol.-% in Stickstoff). Hierzu wurde das Gasgemisch 5 Stunden bei 150°C über den Katalysator geleitet. Im Anschluß wurden 4 g (0,041 mol) Kaliumacetat in 32 ml demineralisiertem Wasser gelöst und portionsweise auf die Katalysatorvorstufe gegeben und diese nochmalig 2 Stunden bei 100°C für 2 Stunden getrocknet.

Der fertige Katalysator enthielt 1,21 Gew.-% Palladium, 0,69 Gew.-% Gold, 2,75 Gew.-% Kalium und 0,06 Gew.-% Vanadium.

### Beispiel 2

0,42 g (0,0016 mol) Vanadylacetylacetonat wurden in 40 ml demineralisiertem Wasser gelöst und auf 65,5 g Trägermaterial aufgebracht. Danach wurde der behandelte Träger 2 Stunden bei 100°C getrocknet.

2,69 g (0,0082 mol) K₂PdCl₄ und 0,96 g (0,0025 mol) KAuCl₄ wurden zusammen eingewogen und in 40 ml demineralisiertem Wasser gelöst. Diese Lösung wurde unter leichter Bewegung auf den vorbehandelten Träger vollständig aufgetragen. Zur Ausbildung einer Edelmetallschale und der Überführung der Edelmetallsalze in unlösliche Verbindungen wurde der vorbehandelte Träger in eine Lösung von 1,91 g (0,034 mol) Kaliumhydroxid in 150 ml demineralisiertem Wasser gegeben und das gesamte Reaktionsgemisch zur Vervollständigung der Reaktion 2,5 Stunden am Rotationsverdampfer bei einer Rotationgeschwindigkeit von 5 rpm bewegt. Die Reaktionsmischung wurde zur Vervollständigung der Reaktion über 14 Stunden ruhengelassen und danach mit demineralisiertem Wasser chloridfrei gewaschen. Die Überprüfung auf Chloridfreiheit erfolgte mittels AgNO₃-Nachweis auf Chloridionen in wässriger Lösung. im Anschluß wurde bei 100°C für 2 Stunden getrocknet.

Anschließend erfolgte die Reduktion der Edelmetalle mit verdünntem Ethylen (5 Vol.-% in Stickstoff). Hierzu wurde das Gasgemisch 5 Stunden bei 150°C über den Katalysator geleitet. Im Anschluß wurden 5 g (0,051 mol) Kaliumacetat in 32 ml demineralisiertem Wasser gelöst und portionsweise auf die Katalysatorvorstufe gegeben und dieser nochmalig 2 Stunden bei 100°C für 2 Stunden getrocknet.

Der fertige Katalysator enthielt 1,21 Gew.-% Palladium, 0,69 Gew.-% Gold, 2,75 Gew.-% Kalium und 0,11 Gew.-% Vanadium.

### Vergleichsbeispiel 1

5,37 g (0,0164 mol) K₂PdCl₄ und 1,92 g (0,005 mol) KAuCl₄ wurden zusammen eingewogen und in 80 ml demineralisiertem Wasser gelöst. Diese Lösung wurde unter leichter Bewegung auf 131 g des Trägermaterials vollständig aufgetragen. Im Anschluß wurde bei 100°C für 2 Stunden getrocknet.

Zur Ausbildung einer Edelmetallschafe und der Überführung der Edelmetallsalze in unlösliche Verbindungen wurde der vorbehandelte Träger mit einer Lösung von 3,81 g (0,068 mol) Kaliumhydroxid in 80 ml demineralisiertem Wasser übergossen. Die Reaktionsmischung wurde zur Vervollständigung der Reaktion über 14 Stunden ruhengelassen und danach mit demineralisiertem Wasser chloridfrei gewaschen. Die Überprüfung auf Chloridfreiheit erfolgte mittels AgNO₃-Nachweis auf Chloridionen in wässriger Lösung. im Anschluß wurde bei 100°C für 2 Stunden getrocknet.

Anschließend erfolgte die Reduktion der Edelmetalle mit verdünntem Ethylen (5 Vol.-% in Stickstoff). Hierzu wurde das Gasgemisch 5 Stunden bei 150°C über den Katalysator geleitet. Im Anschluß wurden 10 g (0,102 mol) Kaliumacetat in 77 ml demineralisiertem Wasser gelöst und portionsweise auf den Katalysator gegeben und dieser nochmalig 2 Stunden bei 100°C für 2 Stunden getrocknet.

Der fertige Katalysator enthielt 1,21 Gew.-% Palladium, 0,69 Gew.-% Gold und 2,75 Gew.-% Kalium.

### Vergleichsbeispiel 2

2,69 g (0,0082 mol) K₂PdCl₄ und 0,96 g (0,0025 mol) KAuCl₄ wurden zusammen eingewogen und in 40 ml demineralisiertem Wasser gelöst. Diese Lösung wurde unter leichter Bewegung auf 65,5 g des Trägermaterials vollständig aufgetragen. Zur Ausbildung einer Edelmetallschale und der Überführung der Edelmetallsalze in unlösliche Verbindungen wurde der vorbehandelte Träger in eine Lösung von 1,91 g (0,034 mol) Kaliumhydroxid in 150 ml demineralisiertem Wasser gegeben und das gesamte Reaktionsgemisch zur Vervollständigung der Reaktion 2,5 Stunden am Rotationsverdampfer bei einer Rotationgeschwindigkeit von 5 rpm bewegt. Die Reaktionsmischung wurde zur Vervollständigung der Reaktion über 14 Stunden ruhengelassen und danach mit demineralisiertem Wasser chloridfrei gewaschen. Die Überprüfung auf Chloridfreiheit erfolgte mittels AgNO₃-Nachweis auf Chloridionen in wässriger Lösung. Im Anschluß wurde bei 100°C für 2 Stunden getrocknet.

Anschließend erfolgte die Reduktion der Edelmetalle mit verdünntem Ethylen (5 Vol.-% in Stickstoff). Hierzu wurde das Gasgemisch 5 Stunden bei 150°C über den Katalysator geleitet. Im Anschluß wurden 4 g (0,041 mol) Kaliumacetat in 32 ml demineralisiertem Wasser gelöst und portionsweise auf den Katalysator gegeben und dieser nochmalig 2 Stunden bei 100°C für 2 Stunden getrocknet.

Der fertige Katalysator enthielt 1,21 Gew.-% Palladium, 0,69 Gew.-% Gold und 2,75 Gew.-% Kalium.

Zur Untersuchung der Leistungsfähigkeit der beschriebenen Katalysatoren in der Herstellung von Vinylacetat wurden Tests in einem Berty-Reaktor und mit einer Feedzusammensetzung von 8,0 Vol.% Sauerstoff, 37,5 Vol.% Ethylen, 15,7 Vol.% Essigsäure und 38,8 Vol.% Stickstoff durchgeführt. Die Ergebnisse sind Tabelle 1 zusammengefaßt:

**Tabelle 1:**

| Katalysatortestungen | | |
|---|---|---|
| Beispiel | Raum-Zeit-Ausbeute | Hochsieder-Selektivität |
| 1 | 683 | 0,5 |
| Vergleichsbeispiel 1 | 688 | 1,1 |
| 2 | 732 | 0,8 |
| Vergleichsbeispiel 2 | 698 | 1,5 |
| Raum-Zeit-Ausbeute in g Vinylacetat/L Katalysator h; Hochsieder-Selektivität in Molprozent, bezogen auf die Menge umgesetzes Ethylen | | |

Wie die Daten aus der obigen Tabelle belegen, führen schon geringe Zusätze an Vanadium zu den bekannten Palladium, Gold und Kalium enthaltenden Katalysatoren zu einer deutlichen Verminderung der Hochsiederbildung in der Vinylacetatherstellung bei gleichzeitiger Steigerung der Leistungsfähigkeit (Raum-Zeit-Ausbeute) der erfindungsgemäßen Katalysatoren.

## Patentansprüche

1. Verfahren zur Herstellung von Vinylacetat in der Gasphase aus Ethylen, Essigsäure und Sauerstoff oder Sauerstoff enthaltenden Gasen an einem Katalysator, der Palladium und/oder dessen Verbindungen, Gold und/oder dessen Verbindungen sowie Alkalimetallverbindungen auf einem Träger enthält, **dadurch gekennzeichnet, daß** der Katalysator zusätzlich 0,01 Gew.-% bis 1 Gew.-% Vanadium und/oder dessen Verbindungen, berechnet als Element und bezogen auf die Gesamtmasse des Katalysators, enthält.

2. Verfahren nach Anspruch 1, **dadurch gekennzeichnet, daß** der Katalysator mindestens eine Kaliumverbindung enthält.

3. Verfahren nach Anspruch 1 oder 2, **dadurch gekennzeichnet, daß** der Katalysator 0,05 Gew.-% bis 0,5 Gew.-% Vanadium, bezogen auf die Gesamtmasse des Katalysators, enthält.

4. Katalysator, der Palladium und/oder dessen Verbindungen, Gold und/oder dessen Verbindungen sowie Alkalimetallverbindungen auf einem Träger enthält, **dadurch gekennzeichnet, daß** der Katalysator zusätzlich 0,01 Gew.-% bis 1 Gew.-% Vanadium und/oder dessen Verbindungen, berechnet als Element und bezogen auf die Gesamtmasse des Katalysators, enthält.

5. Katalysator nach Anspruch 4, **dadurch gekennzeichnet, daß** der Katalysator mindestens eine Kaliumverbindung enthält.

6. Katalysator nach Anspruch 4 oder 5, **dadurch gekennzeichnet, daß** der Katalysator 0,05 Gew.-% bis 0,5 Gew.-% Vanadium, bezogen auf die Gesamtmasse des Katalysators, enthält.

## Claims

1. Process for preparing vinyl acetate in the gas phase from ethylene, acetic acid and oxygen or oxygen-containing gases over a catalyst comprising palladium and/or its compounds, gold and/or its compounds and alkali metal compounds on a support, **characterized in that** the catalyst further comprises from 0.01% by weight to 1% by weight of vanadium and/or its compounds, calculated as element and based on the total mass of the catalyst.

2. Process according to Claim 1, **characterized in that** the catalyst comprises at least one potassium compound.

3. Process according to Claim 1 or 2, **characterized in that** the catalyst contains from 0.05% by weight to 0.5% by weight of vanadium, based on the total mass of the catalyst.

4. Catalyst comprising palladium and/or its compounds, gold and/or its compounds and alkali metal compounds on a support, **characterized in that** the catalyst further comprises from 0.01% by weight to 1% by weight of vanadium and/or its compounds, calculated as element and based on the total mass of the catalyst.

5. Catalyst according to Claim 4, **characterized in that** the catalyst comprises at least one potassium compound.

6. Catalyst according to Claim 4 or 5, **characterized in that** the catalyst contains from 0.05% by weight to 0.5% by weight of vanadium, based on the total mass of the catalyst.

## Revendications

1. Procédé de production d'acétate de vinyle en phase gazeuse à partir d'éthylène, d'acide acétique et d'oxygène ou de gaz contenant de l'oxygène sur un catalyseur qui contient du palladium et/ou ses composés, de l'or et/ou ses composés ainsi que des composés de métaux alcalins sur un support, **caractérisé en ce que** le catalyseur contient en outre 0,01 % en poids à 1 % en poids de vanadium et/ou de ses composés, calculé en élément et rapporté à la masse totale du catalyseur.

2. Procédé selon la revendication 1 **caractérisé en ce que** le catalyseur contient au moins un composé du potassium.

3. Procédé selon la revendication 1 ou 2 **caractérisé en ce que** le catalyseur contient 0,05 % en poids à 0,5 % en poids de vanadium rapporté à la masse totale du catalyseur.

4. Catalyseur qui contient du palladium et/ou ses composés, de l'or et/ou ses composés ainsi que des composés de métaux alcalins sur un support **caractérisé en ce que** le catalyseur contient en outre 0,01 % en poids à 1 % en poids de vanadium et/ou de ses composés calculé en élément et rapporté à la masse totale du catalyseur.

5. Catalyseur selon la revendication 4 **caractérisé en ce que** le catalyseur contient au moins un composé du potassium.

6. Catalyseur selon la revendication 4 ou 5 **caractérisé en ce que** le catalyseur contient 0,05 % en poids à 0,5 % en poids de vanadium rapporté à la masse totale du catalyseur.
